(19)

**Europäisches Patentamt**
**European Patent Office**
**Office européen des brevets**

(11) **EP 4 635 529 A1**

(12) **EUROPEAN PATENT APPLICATION**

(43) Date of publication:
**22.10.2025 Bulletin 2025/43**

(21) Application number: **24170194.5**

(22) Date of filing: **15.04.2024**

(51) International Patent Classification (IPC):
***A61M 5/00*** *(2006.01)*

(52) Cooperative Patent Classification (CPC):
**A61M 5/002; A61M 5/008;** A61M 2005/005;
B65B 3/003

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **SCHOTT Pharma Schweiz AG**
**9001 St. Gallen (CH)**

(72) Inventors:
• **BERLINGER, Marcel**
**9001 St. Gallen (CH)**
• **WALCHER, Ulrich**
**9001 St. Gallen (CH)**

(74) Representative: **Schott Corporate IP**
**Hattenbergstraße 10**
**55122 Mainz (DE)**

(54) **NEST WITH MOVABLE PROTRUSIONS FOR PHARMACEUTICAL CONTAINERS**

(57) A structure comprising a plurality of receptacles, wherein

a. said receptacles are adapted and arranged for receiving a plurality of containers;
b. each receptacle, of the plurality of receptacles, comprises a sidewall defining an interior of a respective receptacle, wherein
i. at least one protrusion is arranged on the sidewall and extends into the interior of the respective receptacle,

ii. the at least one protrusion has a first end and a further end, wherein
A. the first end is arranged opposite the further end,
B. the first end is attached to the sidewall;

wherein at least one section of the further end of the at least one protrusion is adapted and arranged to be movable with respect to the sidewall on which said protrusion is arranged.

Fig. 1      100

EP 4 635 529 A1

**Description**

## FIELD OF THE INVENTION

The invention pertains to a structure for receiving and transporting a plurality of containers, wherein said containers are preferably for accommodating pharmaceutical and/or cosmetic compositions. The invention also pertains to a use of the structure during a filling of a plurality of containers received in the structure. The invention also pertains to a use of the structure for transporting a plurality of containers.

## BACKGROUND

Containers, such as containers for pharmaceutical and/or cosmetic compositions, are generally received (e.g., held) and transported using structures known as nests (such as the nests commercially available from SCHOTT Pharma AG & Co. KGaA). The containers very often have a varying diameter. For example, the container may be a syringe with a cap, wherein a barrel of the syringe has a smaller diameter compared to a diameter of the cap. Therefore, the receptacles in the nest must be dimensioned such that containers with varying diameters can still be received in the nest. However, this leads to the containers tilting in the nest as there is very often a relatively large gap between the barrels and the receptacle sidewalls. Such tilting frequently leads to contact between containers (e.g., between the flanges of syringes), which oftentimes causes damage to said containers. In addition, the tilting of the containers generally leads to problems during filling of said containers with a composition. These problems include varying fill heights of the composition, as well as contact between the containers and the needles used for filling the containers. Such contact can damage the containers, as well as the filling needles. A further problem associated with nests known from the prior art is that a high containers packaging density leads to contact between containers, which damages the containers. Nests in the prior art generally also have a large contact surface between the sidewalls of the receptacles and the containers received in said receptacles. This leads to the formation of undesired particles on the surfaces of the containers.

## OBJECTS

An object of the present invention is to at least partially overcome at least one of the disadvantages encountered in the state of the art.
It is a further object of the invention to provide a structure for receiving a plurality of containers, preferably for accommodating a pharmaceutical and/or cosmetic composition, wherein said structure allows for a higher packaging density of said containers in the structure.
It is a further object of the invention to provide a structure for receiving a plurality of containers, preferably for accommodating a pharmaceutical and/or cosmetic composition, wherein said structure reduces the tilting of the containers when said containers are received in the structure. It is a further object of the invention to provide a structure for receiving a plurality of containers, preferably for accommodating a pharmaceutical and/or cosmetic composition, wherein damage to the containers is reduced when said containers are inserted into and/or removed from the structure.
It is a further object of the invention to provide a structure for receiving a plurality of containers, preferably for accommodating a pharmaceutical and/or cosmetic composition, wherein the structure allows for an easier removal of the containers from the structure.
It is a further object of the invention to provide a structure for receiving a plurality of containers, preferably for accommodating a pharmaceutical and/or cosmetic composition, wherein the structure allows for a reduced damage to said structure during insertion of the containers into the structure.
It is a further object of the invention to provide a structure for receiving a plurality of containers, preferably for accommodating a pharmaceutical and/or cosmetic composition, wherein damage to the containers is reduced when said containers are received in the structure (e.g., during transport of the containers using the structure).
It is a further object of the invention to provide a structure for receiving a plurality of containers, preferably for accommodating a pharmaceutical and/or cosmetic composition, wherein the structure allows for a reduction in the variation of the volumes of a composition in said containers.
It is a further object of the invention to provide a structure for receiving a plurality of containers, preferably for accommodating a pharmaceutical and/or cosmetic composition, wherein the structure allows for a decrease in the downtime of the devices used for at least one or all of the following: inserting the containers in the structure, filling containers received in the structure, removing the containers from the structure.
It is a further object of the invention to provide a structure for receiving a plurality of containers, preferably for accommodating a pharmaceutical and/or cosmetic composition, wherein damage to the containers and/or components used to fill said containers is reduced during a filling of said containers.
It is a further object of the invention to provide a structure for receiving a plurality of containers, preferably for accommodating a pharmaceutical and/or cosmetic composition, that reduces the particle formation on surfaces of said containers when received in the structure.

It is a further object of the invention to provide a structure for receiving a plurality of containers, preferably for accommodating a pharmaceutical and/or cosmetic composition, wherein said containers have a varying diameter.

## PREFERRED EMBODIMENTS OF THE INVENTION

A contribution to at least partially fulfilling at least one of the above-mentioned objects is made by any of the embodiments of the invention.

A 1st embodiment of the invention is a structure comprising a plurality of receptacles, wherein

a. said receptacles are adapted and arranged for receiving a plurality of containers, wherein said containers are preferably for accommodating pharmaceutical and/or cosmetic compositions;
b. each receptacle, of the plurality of receptacles, comprises a sidewall defining an interior of a respective receptacle, wherein

i. at least one protrusion is arranged on the sidewall and extends into the interior of the respective receptacle,
ii. the at least one protrusion has a first end and a further end, wherein

A. the first end is arranged opposite the further end,
B. the first end is attached to the sidewall;

wherein
at least one section of the further end of the at least one protrusion is adapted and arranged to be movable with respect to the sidewall on which said protrusion is arranged.

In a preferred aspect of the 1st embodiment, the receptacles are adapted and arranged for receiving a plurality of containers, wherein each container, of the plurality of containers, has a first section and a further section, and wherein a diameter of the further section is larger than a diameter of the first section. In this aspect, it is preferred that the further section is a body of the container, and the further section is a closing means adapted and arranged to close the container. In this aspect, it is preferred that the first section is a barrel of a syringe, and the further section is a cap of the syringe.

In a preferred embodiment of the structure, at least a segment, preferably the at least one section, of the at least one protrusion, is adapted and arranged to be deformable, preferably elastically deformable.

This preferred embodiment is a 2nd embodiment of the invention, that preferably depends on the 1st embodiment of the invention.

In a preferred embodiment of the structure, the at least one section of the further end is adapted and arranged to rotate through an angle that is in the range from -90° to 90°, preferably from -75° to 75°, and further preferably from -60° to 60°.

This preferred embodiment is a 3rd embodiment of the invention, that preferably depends on any of the 1st to 2nd embodiments of the invention.

In a preferred embodiment of the structure, the at least one protrusion is adapted and arranged to rotate around the first end of the protrusion.

This preferred embodiment is a 4th embodiment of the invention, that preferably depends on any of the 1st to 3rd embodiments of the invention. An example of the 4th embodiment is a protrusion wherein the first end forms a hinge.

In a preferred embodiment of the structure, the first end of the at least one protrusion is adapted and arranged to form a hinge.

This preferred embodiment is a 5th embodiment of the invention, that preferably depends on any of the 1st to 4th embodiments of the invention.

In a preferred embodiment of the structure, the at least one protrusion comprises a polymer selected from the group consisting of polypropylene, polyoxymethylene, polyethylene, polyethylene terephthalate, a polyamide, a polycarbonate, a cyclic olefin copolymer (COC) and a cyclic olefin polymer (COP).

This preferred embodiment is a 6th embodiment of the invention, that preferably depends on any of the 1st to 5th embodiments of the invention. In the 6th embodiment, polypropylene is particularly preferred.

In a preferred embodiment of the structure, a force required to remove a container from the respective receptacle is less than or equal to a weight of the structure, preferably less than the weight of the structure.

This preferred embodiment is a 7th embodiment of the invention, that preferably depends on any of the 1st to 6th embodiments of the invention.

In a preferred embodiment of the structure, the force required to remove a container from the respective receptacle is 1 N or less, preferably 0.5 N or less, more preferably 0.1 N or less, and further preferably 0.075 N or less.

This preferred embodiment is an 8th embodiment of the invention, that preferably depends on any of the 1st to 7th embodiments of the invention.

In a preferred embodiment of the structure, the at least one protrusion has a modulus of elasticity that is in the range 0.80 to 3.50 GPa, preferably from 0.85 to 3.30 GPa, and further preferably from 0.90 to 3.15 GPa, and further preferably from 0.95 to 3.00 GPa.

This preferred embodiment is a 9th embodiment of the invention, that preferably depends on any of the 1st to 8th embodiments of the invention.

In a preferred embodiment of the structure, the respective receptacle has a first end and further end, and wherein the at least one protrusion is arranged closer to the further end than the first end of the respective receptacle.

This preferred embodiment is a 10th embodiment of the invention, that preferably depends on any of the 1st to 9th embodiments of the invention.

In a preferred embodiment of the structure, the respective receptacle has a first end and a further end, and the at least one protrusion is arranged at a distance of $x_1 L$ from the further end of the respective receptacle, where $L$ is the length of the respective receptacle, and $x_1$ is in the range from 0 to 0.5, preferably from 0 to 0.4, more preferably from 0 to 0.3, and further preferably from 0 to 0.2.

This preferred embodiment is an 11th embodiment of the invention, that preferably depends on any of the 1st to 10th embodiments of the invention. In a preferred aspect of the 11th embodiment, the at least one protrusion is arranged at a further end of the respective receptacle (i.e., $x_1 = 0$).

In a preferred embodiment of the structure, the respective receptacle has a first end and a further end, and the at least one protrusion is arranged at a distance from the further end, wherein said distance is in the range from 0 to 8.75 mm, preferably from 0 to 7.00 mm, more preferably from 0 to 5.25 mm, and further preferably from 0 to 3.5 mm.

This preferred embodiment is a 12th embodiment of the invention, that preferably depends on any of the 1st to 11th embodiments of the invention.

In a preferred embodiment of the structure, a distance that the at least one protrusion extends into the interior of the respective receptacle is larger than a maximum thickness of the at least one protrusion.

This preferred embodiment is a 13th embodiment of the invention, that preferably depends on any of the 1st to 12th embodiments of the invention.

In a preferred embodiment of the structure, the at least one protrusion extends a distance $y D_{MIN}$ into the interior volume of the respective receptacle, where $D_{MIN}$ is the minimum diameter of the respective receptacle, where y is in the range from 0.015 to 0.3, preferably from 0.05 to 0.28, more preferably from 0.1 to 0.27, and further preferably from 0.2 to 0.26.

This preferred embodiment is a 14th embodiment of the invention, that preferably depends on any of the 1st to 13th embodiments of the invention.

In a preferred embodiment of the structure, the distance that the at least one protrusion extends into the interior of the respective receptacle is in the range from 0.1 to 9 mm, preferably from 0.3 to 7 mm, even more preferably from 0.4 to 6 mm, and further preferably from 0.5 to 5 mm. This preferred embodiment is a 15th embodiment of the invention, that preferably depends on any of the 1st to 14th embodiments of the invention.

In a preferred embodiment of the structure, a maximum thickness of the at least one protrusion is in the range from 0.05 mm to 4 mm, preferably from 0.1 to 3 mm, more preferably from 0.15 to 2.5 mm, and further preferably from 0.2 to 2.1 mm.

This preferred embodiment is a 16th embodiment of the invention, that preferably depends on any of the 1st to 15th embodiments of the invention. In a preferred aspect of the 16th embodiment, the at least one protrusion has a maximum thickness at the first end of the at least one protrusion.

In a preferred embodiment of the structure, a ratio of a width of the at least one protrusion to a circumference $C$ of the respective receptacle is less than or equal to $0.5C$, preferably less than or equal to $0.25C$, and further preferably less than or equal to $0.1C$.

This preferred embodiment is a 17th embodiment of the invention, that preferably depends on any of the 1st to 16th embodiments of the invention.

In a preferred embodiment of the structure, the at least one protrusion has a width that is less than or equal to 6 mm, preferably less or equal to 5.5 mm, more preferably less than or equal to 5 mm, and further preferably less than or equal to 4.5 mm.

This preferred embodiment is an 18th embodiment of the invention, that preferably depends on any of the 1st to 17th embodiments of the invention. In a preferred aspect of the 18th embodiment, the width is in the range from 0.05 to 6 mm, preferably from 0.1 to 5 mm, and further preferably from 0.15 to 4.5 mm.

In a preferred embodiment of the structure, a length of the at least one protrusion is measured between the first end and the further end of said protrusion, and wherein the thickness of the at least one protrusion, measured at the first end, differs from the thickness of the protrusion, measured at the further end.

This preferred embodiment is an 19th embodiment of the invention, that preferably depends on any of the 1st to

18th embodiments of the invention. In a preferred aspect of the 19th embodiment, the thickness decreases from the first end to the further end. In a preferred aspect of the 19th embodiment, the thickness measured at the first end varies by at least 10 %, more preferably at least 30 %, even more preferably at least 50 %, and further preferably at least 70 %, from the thickness measured at the further end. In a preferred aspect of the 19th embodiment, the thickness measured at the first end varies by less than 150 %, more preferably less than 130 %, even more preferably less than 110 %, and further preferably less than 100 %, from the thickness measured at the further end. In a preferred aspect of the 19th embodiment, the thickness at the first end is 2 mm or less, more preferably 1.8 mm or less, and further preferably 1.6 mm or less. In this aspect, it is preferred that a thickness at the further end is 0.2 mm.

In a preferred embodiment of the structure, a length of the at least one protrusion is measured between the first end and the further end of said protrusion, and wherein the thickness of the at least one protrusion varies by less than 10 %, preferably less than 5 % , and further preferably less than 3 % along the length of the at least one protrusion.

This preferred embodiment is an 20th embodiment of the invention, that preferably depends on any of the 1st to 18th embodiments of the invention.

In a preferred embodiment of the structure, a geometric shape of at least one portion of the at least one protrusion is selected from the group consisting of a flap, a curved plane, and a pin.

This preferred embodiment is a 21st embodiment of the invention, that preferably depends on any of the 1st to 20th embodiments of the invention.

In a preferred embodiment of the structure, the at least one protrusion is adapted and arranged to create a gap between a first segment of the sidewall and a first segment of a container when said container is received (e.g., held) in the respective receptacle.

This preferred embodiment is a 22nd embodiment of the invention, that preferably depends on any of the 1st to 21st embodiments of the invention. In a preferred aspect of the 22nd embodiment, the at least one protrusion is adapted and arranged to create said gap at least at a further end of the respective receptacle.

In a preferred embodiment of the structure, the respective receptacle comprises at least two protrusions, more preferably at least three protrusions, and further preferably three protrusions.

This preferred embodiment is a 23rd embodiment of the invention, that preferably depends on any of the 1st to 22nd embodiments of the invention.

In a preferred embodiment of the structure, the respective receptacle comprises a first set of protrusions, and wherein said protrusions are arranged at positions, as measured parallel to the further direction, that vary by less than 10 %, preferably by less than 7.5 %, and further preferably by less than 5 %.

This preferred embodiment is a 24th embodiment of the invention, that preferably depends on any of the 1st to 23rd embodiments of the invention.

In a preferred embodiment of the structure, the first set of protrusions comprises at least one protrusion, preferably at least two protrusions, more preferably least three protrusions, and further preferably three protrusions.

This preferred embodiment is a 25th embodiment of the invention, that preferably depends on the 24th embodiment of the invention.

In a preferred embodiment of the structure,

a. the respective receptacle comprises a further set of at least one protrusion, and
b. the first set is arranged at a distance from the further set.

This preferred embodiment is a 26th embodiment of the invention, that preferably depends on any of the 24th to 25th embodiments of the invention. In the 26th embodiment, the distance is measured parallel to the further direction, based on the average positions of the protrusions of a set. In a preferred aspect of the 26th embodiment, the further set of protrusions comprises at least two protrusions, more preferably at least three protrusions, and further preferably three protrusions. In the 26th embodiment, if the further set comprises at least two protrusions, said protrusions are arranged at positions, as measured parallel to the further direction, that vary by less than 10 %, preferably by less than 7.5 %, and further preferably by less than 5 %.

In a preferred embodiment of the structure, the respective receptacle has a first end and a further end, and at least one or all of the following applies:

a. the first set protrusions are arranged closer to the further end that the first end;
b. the further set of the at least one protrusion is arranged closer to the first end than the further end.

This preferred embodiment is a 27th embodiment of the invention, that preferably depends on the 26th embodiment of the invention. In an aspect of the 27th embodiment, all possible combination of the features a. and b. are preferred aspects of the embodiment. These combinations are e.g., a; b; a+b.

In a preferred embodiment of the structure, the respective receptacle has a first end and a further end, and at least one or all of the following applies:

a. the first set of protrusions is arranged at a distance of $x_1L$ from the further end of the respective receptacle, where $L$ is the length of the respective receptacle, and $x_1$ is in the range from 0 to 0.5, preferably from 0 to 0.4, more preferably from 0 to 0.3, and further preferably from 0 to 0.2;

b. the further set of the at least one protrusion is arranged at a distance of $x_2L$ from the first end of the respective receptacle, where $L$ is the length of the respective receptacle, and $x_2$ is in the range from 0 to less than 0.5, preferably from 0 to 0.4, more preferably from 0 to 0.3, and further preferably from 0 to 0.2.

This preferred embodiment is a 28th embodiment of the invention, that preferably depends on any of the 26th to 27th embodiments of the invention. In an aspect of the 28th embodiment, all possible combination of the features a. and b. are preferred aspects of the embodiment. These combinations are *e.g.*, a; b; a+b.

In a preferred embodiment of the structure, the sidewall of the respective receptacle has at least one section that has a curved surface, and wherein the at least one section forms at least one protuberance that extends into the interior of the respective receptacle.

This preferred embodiment is a 29th embodiment of the invention, that preferably depends on any of the 1st to 28th embodiments of the invention.

In a preferred embodiment of the structure, the respective receptacle comprises at least two protuberances, and wherein the at least on protrusion is arranged between the at least two protuberances.

This preferred embodiment is a 30th embodiment of the invention, that preferably depends on the 29th embodiments of the invention. In a preferred aspect of the 30th embodiment, if the respective receptacle comprises more than one protrusion, each protrusion is arranged between two protuberances.

In a preferred embodiment of the structure, the at least one protuberance extends along at least 5 %, preferably at least 10 %, more preferably at least 25 %, and further preferably at least 50 % of a length of the respective receptacle.

This preferred embodiment is a 31st embodiment of the invention, that preferably depends on any of the 29th to 30th embodiments of the invention.

In a preferred embodiment of the structure, the respective receptacle comprises at least two, preferably at least four, and more preferably at least six protuberances.

This preferred embodiment is a 32nd embodiment of the invention, that preferably depends on any of the 29th to 31st embodiments of the invention.

In a preferred embodiment of the structure, a diameter of the interior varies along a length of the respective receptacle, preferably wherein a first diameter of the interior, measured at a first end of the respective receptacle, is larger than a further diameter, measured at a further end of the respective receptacle.

This preferred embodiment is a 33rd embodiment of the invention, that preferably depends on any of the 1st to 32nd embodiments of the invention.

In a preferred embodiment of the structure, the structure further comprises a plurality of containers that contain less than 0.1 ml, preferably less than 0.05 ml, and further preferably less than 0.01 ml of a composition.

This preferred embodiment is a 34th embodiment of the invention, that preferably depends on any of the 1st to 33rd embodiments of the invention.

In a preferred embodiment of the structure, the structure further comprises a plurality of containers that are at least 20 %, preferably at least 35 %, and more preferably at least 50 %, filled with a composition, wherein said percentages are with respect to an interior volume of a container.

This preferred embodiment is a 35th embodiment of the invention, that preferably depends on any of the 1st to 34th embodiments of the invention.

In a preferred embodiment of the structure, each container, of the plurality of containers, has a first section and a further section, wherein a diameter of the further section is larger than a diameter of the first section.

This preferred embodiment is a 36th embodiment of the invention, that preferably depends on the 35th embodiment of the invention. In a preferred aspect of the 36th embodiment, the further section is a body of the container, and the further section is a closing means adapted and arranged to close the container. In a preferred aspect of the 36th embodiment, the first section is a barrel of a syringe, and the further section is a cap of the syringe.

A 37th embodiment of the invention is a method for at least partially filling a plurality of containers with a composition, preferably a pharmaceutical and/or cosmetic composition, comprising the steps of

a. providing a structure according to the invention, preferably the structure according to any of the 1 to 33rd embodiments of the invention,

b. inserting a plurality of containers in the plurality of receptacles of the structure, wherein the at least one protrusion preferably deforms during said insertion, and wherein the containers are preferably according to any of the 34th to 36th embodiments of the invention;

c. at least partially filling the containers with the composition.

In a preferred aspect of the 37th embodiment, each container, of the plurality of containers, has a first section and a further section, wherein a diameter of the further section is larger than a diameter of the first section. In this aspect, it is preferred that the further section is a body of the container, and the further section is a closing means adapted and arranged to close the container. In this aspect, it is preferred that the first section is a barrel of a syringe, and the further section is a cap of the syringe.

In a preferred embodiment of the method for at least partially filling a plurality of containers with a composition, the method comprises the following further step:

d. removing the plurality of containers from the structure, wherein the at least one protrusion preferably deforms during said removal.

This preferred embodiment is a 38th embodiment of the invention, that preferably depends on the 37th embodiment of the invention.

A 39th embodiment of the invention is a plurality of containers at least partially filled with a composition, obtainable by a method (according to the invention) for filling a plurality of containers with a composition, preferably the method according to any of the 37th to 38th embodiments of the invention.

A 40th embodiment of the invention is a method for transporting a plurality of containers, comprising the steps of

a. providing a structure according to the invention, preferably the structure according to any of the 1 to 33rd embodiments of the invention;
b. inserting a plurality of containers in the structure, wherein the containers are preferably according to any of the 34th to 36th embodiments of the invention;
c. transporting the structure and the plurality of containers from a first location to a further location.

In a preferred aspect of the 40th embodiment, each container, of the plurality of containers, has a first section and a further section, wherein a diameter of the further section is larger than a diameter of the first section. In this aspect, it is preferred that the further section is a body of the container, and the further section is a closing means adapted and arranged to close the container. In this aspect, it is preferred that the first section is a barrel of a syringe, and the further section is a cap of the syringe.

In a preferred aspect of the method for transporting a plurality of containers, the method further comprises at least one or all of the following steps:

A. inserting the structure into an even-further structure that at least partially encloses the structure, e.g., a tub;
B. sealing an opening of the even-further structure with a film, preferably a gas permeable film;
C. sterilising the structure and the containers received therein, preferably while sealed in an even-further structure, e.g., the tub.

This preferred embodiment is a 41st embodiment of the invention, that preferably depends on the 40th embodiment of the invention. In an aspect of the 41st embodiment, all possible combination of the features A. to C. are preferred aspects of the embodiment. These combinations are *e.g.,* A; B; C; A+B; A+C; B+C; A+B+C. In the 41st embodiment, a "tub" should be understood as used in the pharmaceutical industry. In a preferred aspect of the 41st embodiment, one or more of the steps A. to C. are performed at the first location, while one or more of the steps A. to C. are performed at the further location. For example, steps A. and B. are performed at the first location after performing step b. in the 40th embodiment, the structure with the containers are then transported to the further location (step c. in 40th embodiment), where step C. is then performed at the further location.

A 42nd embodiment of the invention is a use of a structure according to the invention, preferably the structure according to any of the 1 to 36th embodiments of the invention, for at least one or all of the following:

a. reducing contact between the plurality of receptacles and a plurality of containers received in said receptacles, preferably reducing contact between the plurality of receptacles and the further ends of the plurality of containers received in said receptacles;
b. reducing contact between neighbouring containers received in the structure;
c. reducing a particle count on the surfaces of the containers received in the structure;
d. reducing damage to the containers when received in the structure.

In an aspect of the 42nd embodiment, all possible combination of the features a. to d. are preferred aspects of the embodiment. These combinations are *e.g.,* a; b; c; d; a+b; a+c; a+d; b+c; b+d; c+d; a+b+c; a+b+d; a+c+d; b+c+d; a+b+c+d.

## DETAILED DESCRIPTION OF THE INVENTION

Features described as preferred in one category of the invention, for example according to the structure, are analogously preferred in an embodiment of the other categories according to the invention, such as a use of the structure according to the invention.

Throughout this document, disclosures of ranges should preferably be understood to include both end points of the range. Furthermore, each disclosure of a range in the document should preferably be understood as also disclosing preferred sub-ranges in which one end point is excluded or both end points are excluded. For example, a disclosure of a range from $X_1$ to $X_2$ is to be understood as disclosing a range that includes both of the end points $X_1$ and $X_2$. Further-more, it is to be understood as also disclosing a range that includes the end point $X_1$ but excludes the end point $X_2$, a range that excludes the end point $X_1$ but includes the end point $X_2$, and a range that excludes both end points $X_1$ and $X_2$.

Some preferred embodiments and preferred aspects have various combinations of features as alternatives. If the various combinations are disclosed, these combinations are separated by a semi-colon (";"). For example, the list of features "a; a+b; a+c+d" for a preferred embodiment discloses a preferred embodiment that comprises the feature "a", a preferred embodiment that comprises the features "a" and "b", and a preferred embodiment that comprises the features "a", "c", and "d".

In an embodiment of the invention, the structure "comprises a plurality of receptacles, wherein each receptacle, of the plurality of receptacles, comprises a sidewall defining an interior of a respective receptacle, wherein the sidewall comprises at least one protrusion arranged on the sidewall and extending into the interior of the respective receptacle; wherein the at least one protrusion has a first end and a further end, wherein the first end is arranged opposite the further end, and the first end is attached to the sidewall; wherein at least one section of the further end of the at least one protrusion is adapted and arranged to be movable with respect to the sidewall on which said protrusion is arranged". This should not be understood to mean that the structure can only have receptacles with protrusion that have at least one section that is moveable with respect to a receptacle sidewall. The structure may, in addition to the aforementioned plurality of receptacles, e.g., further comprise a further plurality of receptacles, wherein the sidewalls of the receptacles (of the further plurality of receptacles) do not comprise protrusions or wherein the protrusions (of the further plurality of receptacles) do not have a movable further end.

In an aspect of the invention, it is preferred that at least 70 %, more preferably at least 85 %, even more preferably at least 95 %, and further preferably all of the receptacles of the first structure form part of the plurality of receptacles (i.e., having sidewalls comprising at least one protrusion that has at least one section that is movable with respect to a receptacle sidewall). Preferred embodiment and preferred aspects of "the respective receptacle" should be understood to be preferred embodiment and preferred aspects of at least 70 %, more preferably at least 85 %, even more preferably at least 95 %, and further preferably all of the receptacles that form the plurality of receptacles.

An example of a container received in a receptacle is a container that is held in said receptacle.

### Structure

A structure preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for the structure.

A structure may be obtained using deep drawing moulding, preferably injection moulding, and/or 3D printing. A structure is preferably manufactured as a single piece. A structure for receiving containers, wherein said containers are for accommodating pharmaceutical and/or cosmetic compositions, is often referred to as a "nest". Here "nest" should preferably be understood as used in the pharmaceutical industry.

A structure preferably comprises 5 to 250, more preferably 10 to 200, even more preferably 20 to 190, further preferably 40 to 180, further preferably 60 to 180, further preferably 80 to 180, and even further preferably 100 to 160, receptacles.

A structure preferably has a mass in the range from 40 to 400 g, more preferably from 55 to 250 g, and further preferably from 75 to 100 g.

In one preferred aspect of the invention, at least portions of the sidewalls of neighbouring receptacles do not touch (e.g., a gap is formed between said portions). In another preferred aspect of the invention, the sidewalls of neighbouring receptacles touch. In another preferred aspect of the invention, neighbouring receptacles share at least a portion of the same sidewall.

### Receptacle

A receptacle preferably extends from a first surface of the structure. A preferred receptacle is elongated. A preferred receptacle has a first end and a further end, opposite the first end. A first end of a receptacle preferably has a first opening for receiving a container. In other words, a receptacle is preferably adapted and arranged for a container to be inserted into and/or removed from said respective receptacle at the first end. Preferably, the further end of a receptacle has a further opening. A further opening at the further end of a receptacle is preferably adapted and arranged to allow a part of a container to extend out of the receptacle when said container is received in the receptacle.

### Protrusion

A protrusion preferably comprises, more preferably is made of, at least one thermoplastic. A cross-section of a protrusion may any geometric shape, wherein said cross-section is made perpendicular to a length of the protrusion. For example, if a protrusion is in the form of a pin, said pin can have a cylindrical or rectangular cross-section.

In a preferred aspect of the invention, a protrusion is formed as one piece with the sidewall of a receptacle. In a preferred aspect of the invention, a protrusion extends in a direction that is substantially perpendicular to the sidewall of the receptacle. In one preferred aspect of the invention, a protrusion and the sidewall, on which said protrusion is arranged, is made from the same material. In another preferred aspect of the invention, a protrusion and the sidewall, on which said protrusion is arranged, are made from different materials.

A set of protrusions should preferably be understood as one or more protrusions that are arranged at positions, as measured parallel to the further direction, that vary by less than 10 %. A set may comprise only one protrusion.

A protrusion that has a geometric shape in the form of a flap should preferably be understood to mean that the protrusion comprises at least one first portion, wherein a maximum width of the at least one first portion is larger than a maximum thickness of said at least one first portion. A protrusion may also comprise at least two of the aforementioned first portions, wherein said at least two aforementioned first portions may be arranged at an angle with respect to each other. A protrusion may also comprise one or more further portions in the shape of a curved plane. A A protrusion may also comprise two or more of the aforementioned further portions that are arranged at an angle with respect to each other.

### Movable and Deformable

A section of a further end of a protrusion, that is adapted and arranged to be moveable with respect to a sidewall of a receptacle, should be understood to mean that the further end of the protrusion can move while the sidewall, on which said protrusion is arranged, remains stationary.

A section of a further end of a protrusion, that is adapted and arranged to be moveable with respect to a sidewall of a receptacle, should be understood to mean that said section can move without damaging the protrusion. An example of damaging a protrusion is the breaking off, or the tearing off, of the further end of the protrusion.

A protrusion that is adapted and arranged to be deformable should preferably be understood to mean that at least one or all of the following: upon application of a force on the protrusion, a a further end of the protrusion can move with respect to the sidewall without breaking said protrusion; upon application of a force on the protrusion, a shape of the protrusion can be changed without breaking said protrusion. For example, the protrusion may be bent along a length of the protrusion when a container in inserted into a receptacle, and the containers comes into contact with the protrusion.

A protrusion that is adapted and arranged to be elastically deformable should preferably be understood as follows: a further end of a protrusion is located at a first position when no force is applied to the protrusion (e.g., the protrusion is a pin that is straight). Upon application of a force, the further end of the protrusion moves to a further position (e.g., the pin is bent). Upon removal of the force, the further end of the protrusion at least partially returns to the first position (e.g., the protrusion becomes straighter, but may still be bent).

### Force

In the context of the present disclosure, a force applied on a protrusion should preferably be understood to have a magnitude of the same order of magnitude as the force that is exerted, by a container, when said container is inserted into and/or removed from a receptacle.

### Angle of rotation

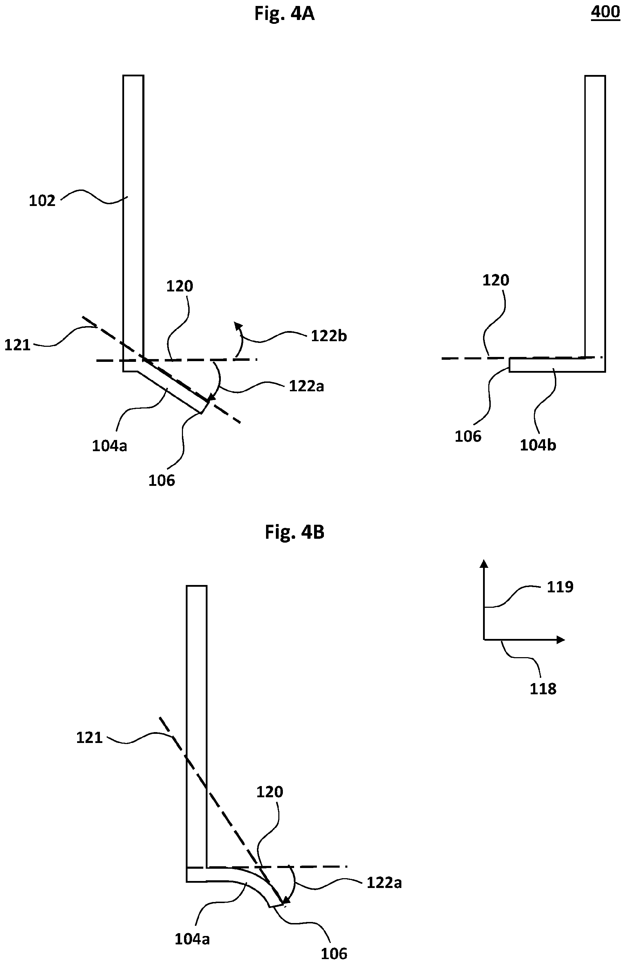

The angle of rotation of at least a section of a further end of a protrusion is measured with respect to a plane that defines 0°. This plane is the neutral plane, as illustrated in Figs 4A and 4B. The further end lies in said plane when no forces are being applied to the further end.

### Protuberance

In one preferred embodiment of the invention, the sidewall of the respective receptacle has at least one section that has a curved surface, and wherein the at least one section forms at least one protuberance that extends into the interior of the respective receptacle". This feature is referred herein as "the sidewall comprises a protuberance".

A protuberance preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for a protuberance. A protuberance is preferably formed as one piece with the sidewall of a receptacle. In a preferred aspect of the invention, a protuberance extends in a direction that is substantially perpendicular to the sidewall of the receptacle.

The feature "the sidewall of the respective receptacle has at least one section that has a curved surface, wherein the at least one section forms at least one protuberance" should be understood to mean that the protuberance has a curved surface. A curvature, of a surface of a section of the sidewall forming a protuberance, is preferably perpendicular to a length of a respective receptacle.

A protuberance preferably extends along at least 75 %, more preferably at least 85 %, and further preferably at least 95 % of a length of the receptacle wherein said protuberance is located.

If a receptacle comprises protuberances, it is preferred that said receptacle comprises an even number of protuberances.

Containers

A container as referred herein comprises a container wall at least partially enclosing an interior volume of said container. A container as referred to herein is designed to accommodate a composition in such a way that an inner side of the container wall (i.e., the side of the container wall which faces the interior volume) and/or a coating layer (e.g., lubrication layer) on the inner side of the container wall can be in direct contact with the composition. A preferred container is suitable to be used for accommodating a pharmaceutical and/or cosmetic composition.

A first end of a container preferably comprises a first orifice, which allows for the charging of a composition into the interior volume of the container. Preferably, the first orifice also allows for the discharging of a composition from the interior volume.

An interior volume of a container represents the full volume of the interior of the container. This volume may be determined by filling the interior volume with water up to the brim of the container and measuring the volume of the amount of water which the interior volume can take up to the brim. Hence, the interior volume as used herein is not a nominal volume as it is often referred to in the technical field of pharmacy. This nominal volume may for example be less than the interior volume.

A container may have any size or shape which the skilled person deems appropriate in the context of the invention. A preferred container has at least one section that is cylindrical. A preferred container is elongated.

A preferred container, more preferably the container wall, comprises, more preferably consists of, at least one glass, at least one polymer, or a combination thereof. A glass may be any type of glass and may have any composition which the skilled person deems suitable in the context of the invention. Preferably, the glass is suitable for pharmaceutical compositions. A preferred glass is of type I in accordance with the definitions of glass types in section 3.2.1 of the European Pharmacopoeia, 7th edition from 2011. Examples of a preferred glass include borosilicate glass, fused silica, and combinations thereof. A particularly preferred glass is a borosilicate glass. Examples of a preferred polymer include polypropylene, polyoxymethylene, polyethylene, a cyclic olefin copolymer (COC) and a cyclic olefin polymer (COP). Here a cyclic olefin copolymer (COC) and a cyclic olefin polymer (COP) are particularly preferred.

Examples of preferred containers include cartridges, vials, ampules, carpules, and syringes. Preferably, all of the containers, of the plurality of containers, are of the same type of container, which is preferably selected from the above-mentioned mentioned examples of preferred containers. A preferred syringe is made from glass or polymer.

Additionally or alternatively, a preferred syringe has an interior volume in the range from 0.5 to 100 ml, preferably from 0.5 to 80 ml, more preferably from 0.5 to 60 ml. Additionally or alternatively, a preferred syringe comprises a body part which is preferably cylindrical. This is also referred to as barrel. A preferred barrel of a syringe has a diameter in the range from 5 to 50 mm, more preferably from 6 to 45 mm, even more preferably from 8 to 40 mm, most preferably from 9 to 35 mm. Particularly preferable, the barrel is made from a glass or a polymer.

A preferred container, more preferably the container wall, more preferably a surface of the container wall facing the interior volume of the container, comprises at least one coating layer. Here a coating layer should preferably be understood as a layer that is applied by any method known to a skilled person working in the pharmaceutical industry. These methods include the application of a coating layer by spraying or wiping said coating layer on the container wall facing the interior volume of the container. A preferred coating layer is a lubrication layer. A preferred lubrication layer comprises silicon. A preferred lubrication layer reduces friction when a component of the container is moved in the interior volume of the container. Here an example of a component is a plunger of a syringe, with the syringe being an example of a container. For example, a lubrication layer reduces the force required to move a plunger in a barrel of a syringe.

Pharmaceutical and cosmetic composition

In the context of the invention, every pharmaceutical composition and every cosmetic composition which the skilled person deems suitable comes into consideration. A pharmaceutical composition is a composition comprising at least one pharmaceutically active ingredient. A preferred pharmaceutically active ingredient is a vaccine. A cosmetic composition is a composition comprising at least one cosmetically active ingredient. A preferred cosmetically active ingredient is hyaluronic acid or botulinum toxin. The pharmaceutical and/or cosmetic composition may be fluid or solid or both, wherein a fluid composition is particularly preferred herein. A preferred solid composition is granular such as a powder, a multitude of tablets or a multitude of capsules. A further preferred pharmaceutical and/or cosmetic composition is a parenterialium, i.e., a composition which is intended to be administered via the parenteral route, which may be any route which is not enteral. Parenteral administration can be performed by injection, e.g. using a needle (usually a hypodermic needle) and a syringe, or by the insertion of an indwelling catheter.

The invention is now illustrated by non-limiting examples and exemplifying embodiments.

## FIGURES

List of figures

The figures serve to exemplify the present invention and should not be viewed as limiting the invention. Furthermore, the figures are not drawn to scale.

Fig. 1: schematic illustration showing a cross-section of a structure according to the invention.
Figs 2A and 2B: schematic illustrations showing a further end of protrusion being movable with respect to a receptacle sidewall.
Figs 3A to 3D: schematic illustrations showing how the dimensions of a receptacle and protrusion are measured.
Figs 4A and 4B: schematic illustrations showing how a rotation angle of a protrusion is measured.
Figs 5A to 5C: illustrations of a first embodiment of a protrusion according to the invention.
Figs 6A to 6C: illustrations of a second embodiment of a protrusion according to the invention.
Figs 7A to 7C: illustrations showing a first and further set of protrusions according to the invention.
Fig. 8: flow diagram showing the steps of a method 800 for filling a plurality of containers.
Fig. 9: flow diagram showing the steps of a method for a method for transporting a plurality of containers.
Fig. 10: schematic illustration of the test method used to measure the force required to remove a container from a receptacle.
Fig. 11: example of a first end of a protrusion that is formed as a hinge.

Description of figures

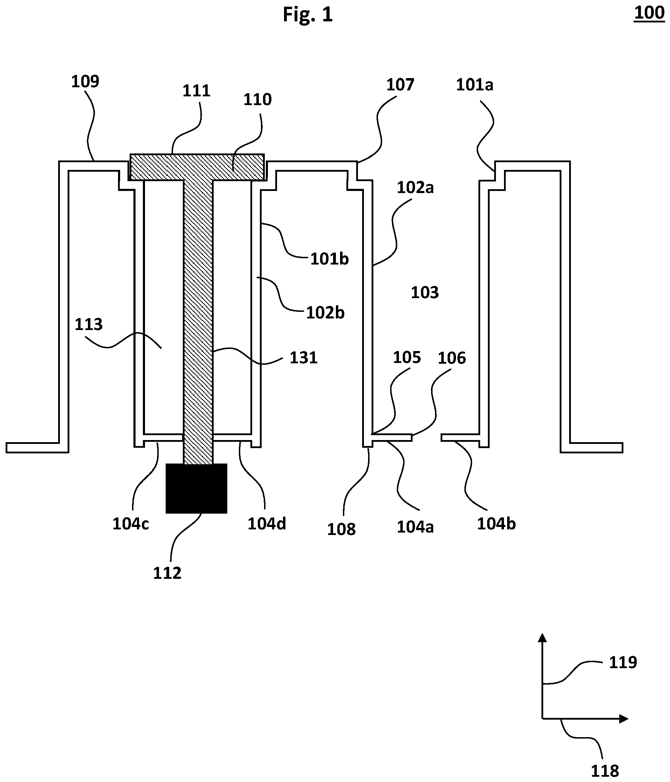

Fig. 1 is a schematic illustration showing a cross-section of a structure 100 according to the invention. The structure 100 is viewed from the side. The structure has a plurality of receptacles, such as receptacles 101a. As shown for receptacle 101a, a receptacle has a sidewall 102a defining an interior 103 of said receptacle 101a. Protrusions 104a and 104b are arranged on the sidewall 102a, with said protrusions 104a and 104b extending into the interior 103 of receptacle 101a. As shown for protrusion 104a, a protrusion has a first end 105 and a further end 106 (arranged opposite the first end 105), with the first end 105 attached to the sidewall 102a. As also shown for receptacle 101a, a receptacle has a first end 107 and a further end 108. The first end 107 is located at, or near, a first surface 109 of the structure 100. The first surface 109 may also be referred to as the top or upper surface of the structure 100. Fig. 1 further shows that a container 110 is received in the receptacle 101b. Said container 110 has a first end 111 and a further end 112, as well as a body 131. It can be seen from Fig. 1 that the body 131 (an example of a first section) of the container 110 has a smaller diameter than the diameter of the further end 112 (an example of a further section). As a result, a gap 113 is formed between the container 110 and a sidewall 102b of the receptacle 101b. Protrusions 104c and 104d are in contact with the container 110, thereby at least partially inhibiting the container 110 tilting in the receptacle 101b. Preferably the contact between the protrusions 104c and 104d and the container 110 is such that the container 110 is not gripped by said protrusions. The container 110 in Fig. 1 is a syringe with a flange (the first end 111), a barrel (the body 131), and a cap (the further end 112). A container is inserted into the structure 100 at a first end of a receptacle.

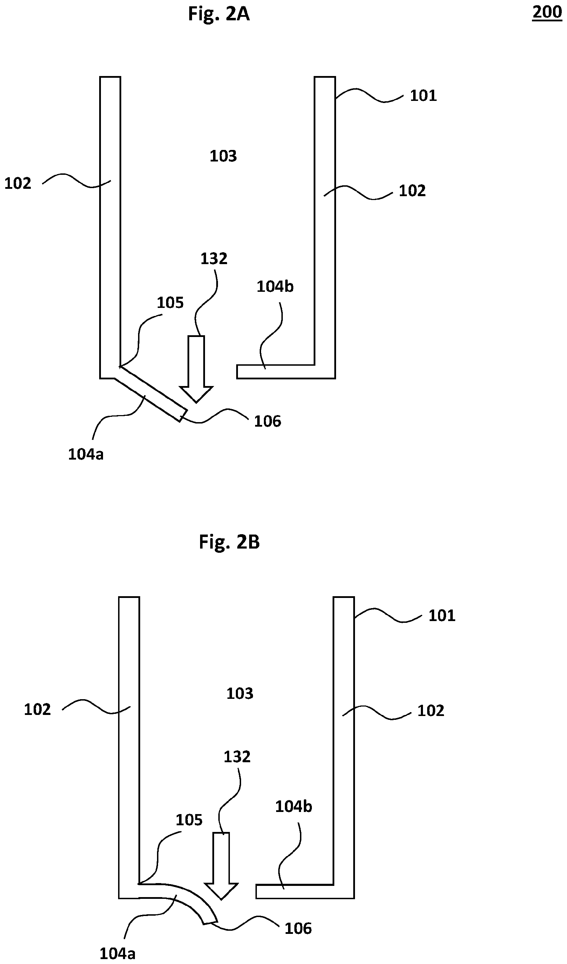

Figs 2A and 2B are schematic illustrations showing a further end of protrusion being movable with respect to a receptacle sidewall. In particular, Figs 2A and 2B show receptacles 101 of a structure according to the invention. Fig. 2A shows a force 132 being applied to the protrusion 104a. This force 132 results from, e.g., a container (not shown) being inserted into the receptacle, and a further end of said container being in contact with the protrusion 104a. As a result, a further end 106 of the protrusion 104a moves with respect to a sidewall 102 of the receptacle 101 (e.g., the protrusion 104a is deformed). More specifically, the protrusion 104a rotates around a first end 105 of the protrusion 104a (e.g., the first end 105 is formed as a hinge). For comparison, protrusion 104b is shown, with no force being applied to protrusion 104b. It can be seen that protrusion 104b is positioned perpendicular to the sidewall 102. This position, when no force is being applied, is also referred to as the neutral position of a protrusion. It is preferred that if a further end of a protrusion moved with respect to a sidewall, during insertion and/or removal of a container, that the protrusion at least partially returns to the neutral position once said container is positioned in and/or has been removed from the receptacle (i.e., the protrusion is elastically deformable). It may be noted that when a container is inserted into a receptacle, the force will very likely be applied to all protrusions in said receptacle. However, for the purposes of illustration, protrusion 104b is shown without a force being applied.

Fig. 2B is the same as Fig. 2A, with the following difference. As can be seen from Fig. 2B, the protrusion 104a bends along a length of said protrusion during the application of the force 132 (e.g., the protrusion 104a is deformable). In Fig. 2B the first end 105 of the protrusion 104a is not formed as a hinge. However, it should be noted that a protrusion may deform as shown in Fig. 2B, even if the first end 105 of the protrusion 104a is formed as a hinge.

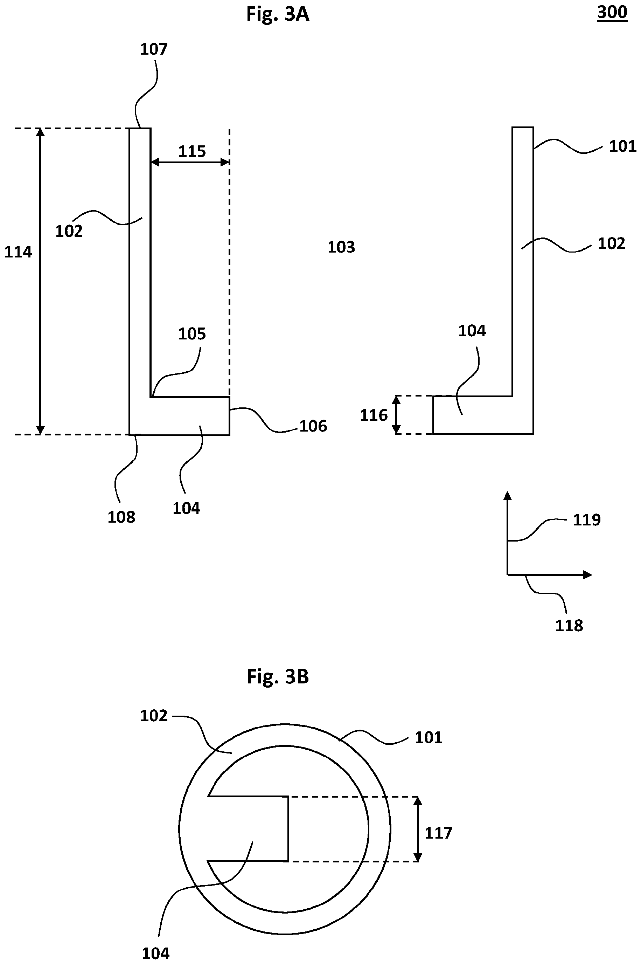

Figs 3A to 3D are schematic illustrations showing how the dimensions of a receptacle and protrusion are measured. For the purposes of illustration, the receptacles are only shown partially. Fig. 3A shows that a length 114 of a receptacle 101 is measured between the first end 107 and further 108 end of said receptacle. The length 114 is also measured parallel to a further direction 119. This also applies when a receptacle has a varying diameter (e.g., the receptacle has a conical shape). Here the length is measured along an axis that passes through a centre of the receptacle, with said axis parallel to the further direction 119. A distance 115 that a protrusion extends into an interior 103 of a receptacle 101 is measured between the first end 105 and the further end 106 of a protrusion 104. Furthermore, the distance 115 is measured parallel to a first direction 118, wherein the first direction 118 is perpendicular to the further direction 119. If a receptacle has a varying diameter (e.g., the receptacle has a conical shape), the distance 115 is nevertheless measured parallel to the first direction 118 (i.e., the distance is not measured along an axis that is perpendicular to the sidewall 102, as the sidewalls are not parallel to the further direction 119). A thickness 116 of a protrusion 104 is measured as shown in Fig. 3A. Fig. 3B is a top view of a receptacle 101. A width 117 of a protrusion 104 is measured as shown in Fig. 3B. It may be noted that the thickness 116 and the width 117 are not necessarily measured parallel to the first direction 118, the further direction 119, or a direction that is perpendicular to both the first 118 and further 119 directions.

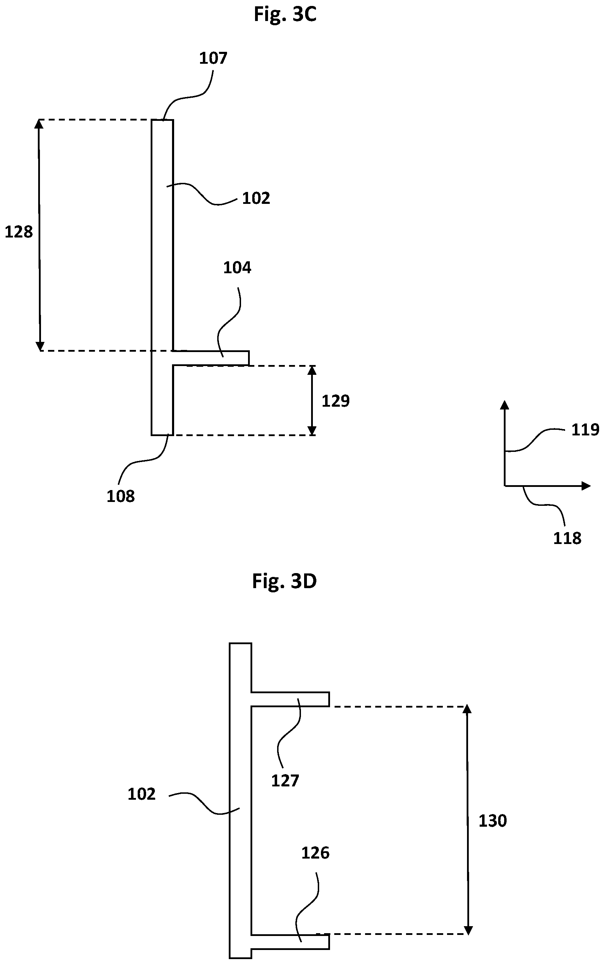

Fig. 3C shows how a distance 128 of a protrusion 104 from a first end 107 of a receptacle is measured. Fig. 3C also shows how a distance 129 of a protrusion 104 from a further end 108 of a receptacle is measured. Both of these distances are measured parallel to the further direction 119, even if a receptacle has a varying diameter (e.g., the receptacle has a conical shape). Fig. 3D shows how a distance between a protrusion, of a first set 126 of protrusions, and a protrusion, of a further set 127 of protrusions, is measured. Said distance is measured parallel to the further direction 119, even if a receptacle has a varying diameter (e.g., the receptacle has a conical shape).

Figs 4A and 4B are schematic illustrations showing how a rotation angle of a protrusion is measured. For the purposes of illustration, the receptacles are only shown partially. Fig. 4A corresponds to the receptacle shown in Fig. 2A. When a protrusion is in the neutral position (such as protrusion 104b), the further end 106 of the protrusion 104b lies in a neutral plane 120. Upon application of a force of sufficient magnitude, the further end 106 of the protrusion 104a moves out of the neutral plane 120. A plane 121, that is tangential to the further end 106, is fitted to the protrusion 104a. The angle 112a between the neutral plane 120 and the tangential plane 121 is defined as the angle of rotation of the further end 106. Fig. 4B corresponds to the receptacle shown in Fig. 2B. The angle of rotation of the further end 106 in Fig. 4B is determined in the same manner as described for Fig. 4A. It may be noted that the angle of rotation need not be only around a direction that is perpendicular to the first direction 118 and the further direction 119. If a protrusion is rotated around, e.g., the further direction 119, the neutral plane in Figs 4A and 4B should be accordingly rotated around the first direction 118.

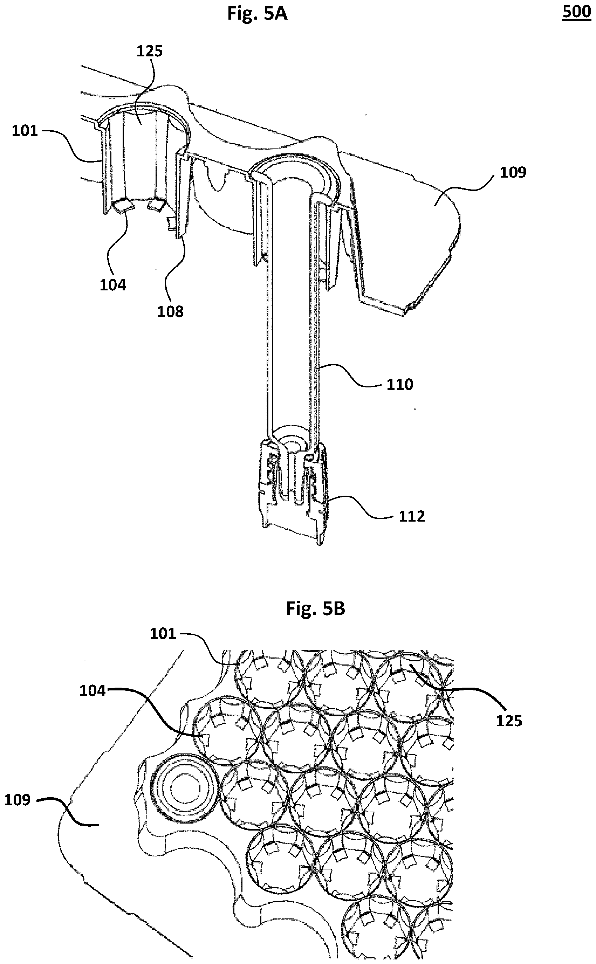

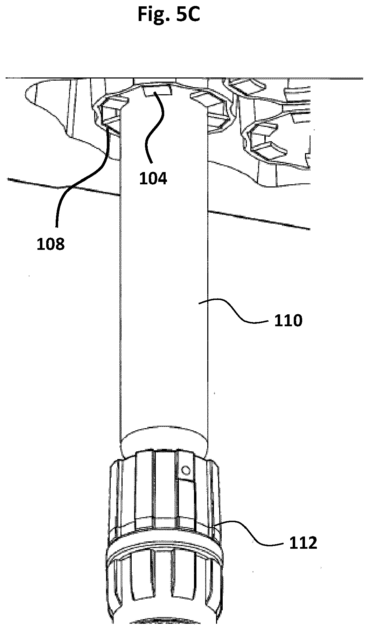

Figs 5A to 5C are illustrations of a first embodiment of a protrusion according to the invention. Fig. 5A is an isometric view of a cross-section of a structure according to the invention. As can be seen, the protrusions 104 are arranged as flaps at the further end 108 of receptacle 101. The receptacle 101 also has protuberances 125 extending along a length of the receptacle 101. The protrusions 104 are arranged between the protuberances 125. Fig. 5B is a top view of the structure of Fig. 5A, where it can be seen that the protrusions 104 are arranged around the circumference of the receptacles 101. Fig. 5C is a view from below of the structure in Fig. 5A. Here it may be noted that the protrusions 104 are in a neutral position when a container 110 is received in a receptacle.

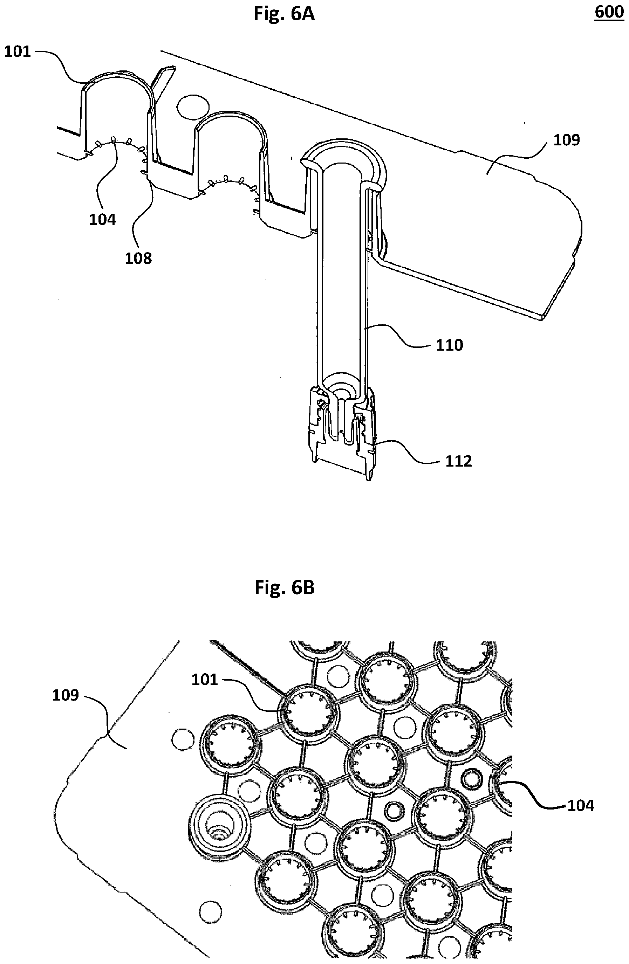

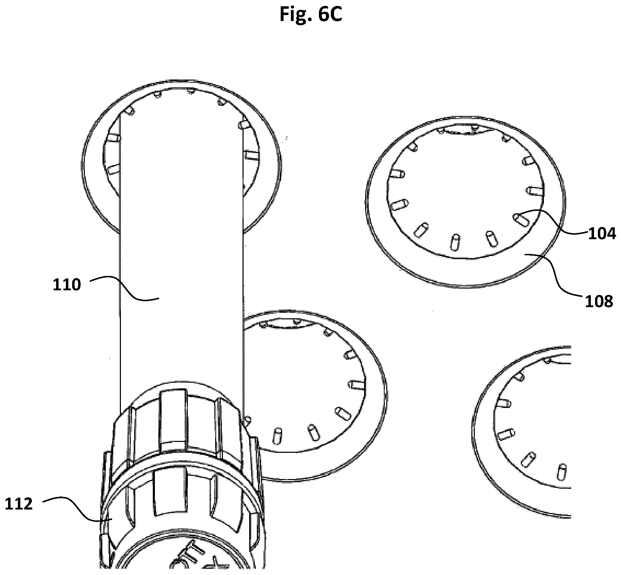

Figs 6A to 6C are illustrations of a second embodiment of a protrusion according to the invention. Fig. 6A is an isometric view of a cross-section of a structure according to the invention. As can be seen, the protrusions 104 are arranged as pins at a distance from the further end 108 of receptacle 101. Fig. 6B is a top view of the structure of Fig. 6A, where it can be seen that the protrusions 104 are arranged around the circumference of the receptacles 101. Fig. 6C is a view from below of the structure in Fig. 6A. Here it may be noted that the protrusions 104 are in a neutral position when a container 110 is received in a receptacle.

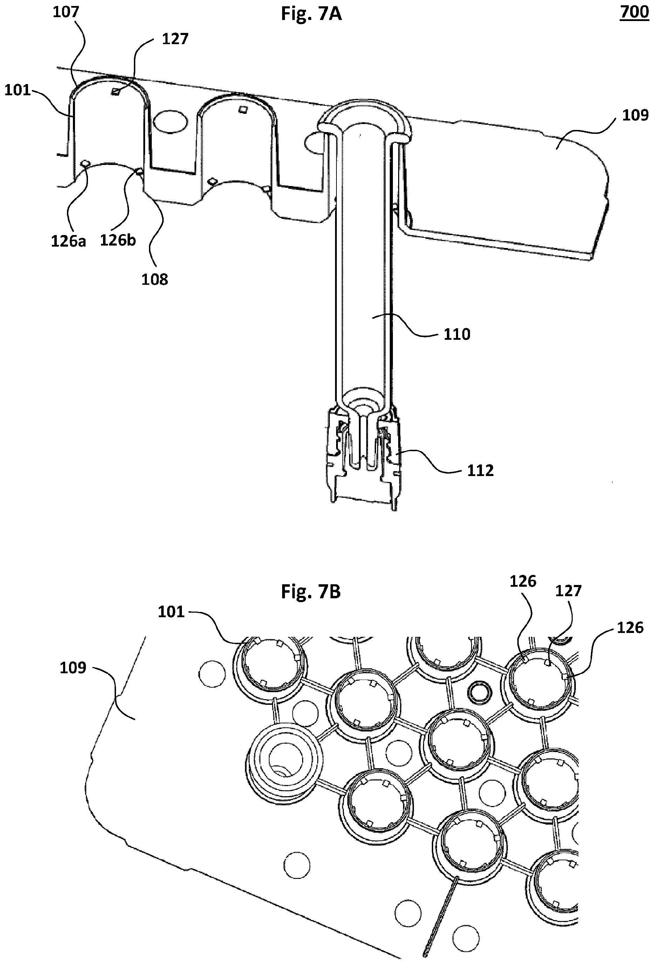

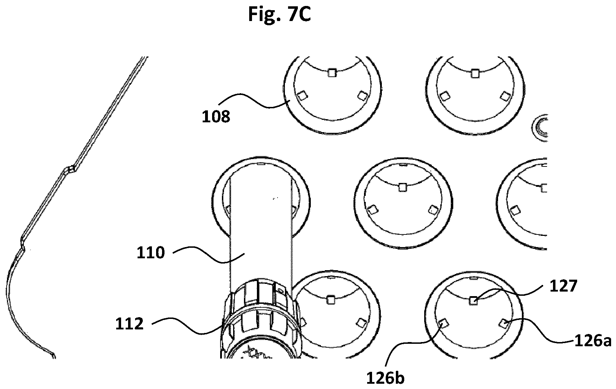

Figs 7A and 7C are illustrations showing a first set and a further set of protrusions according to the invention. Fig.

7A is an isometric view of a cross-section of a structure according to the invention. The receptacle 101 has a first set 126 of protrusion (protrusions 126a and 126b are shown) that are arranged closer to the further end 108 of the receptacle 101 than the first end 107. The receptacle 101 also has a further set 127 of protrusions that are arranged closer to the first end 107 of the receptacle 101 than the further end 108. Fig. 6B is a top view of the structure of Fig. 6A, where it can be seen that the protrusions of the first set 126 and the further set 127 are arranged alternatingly around a circumference of a receptacle 101. Fig. 6C is a view from below of the structure in Fig. 6A.

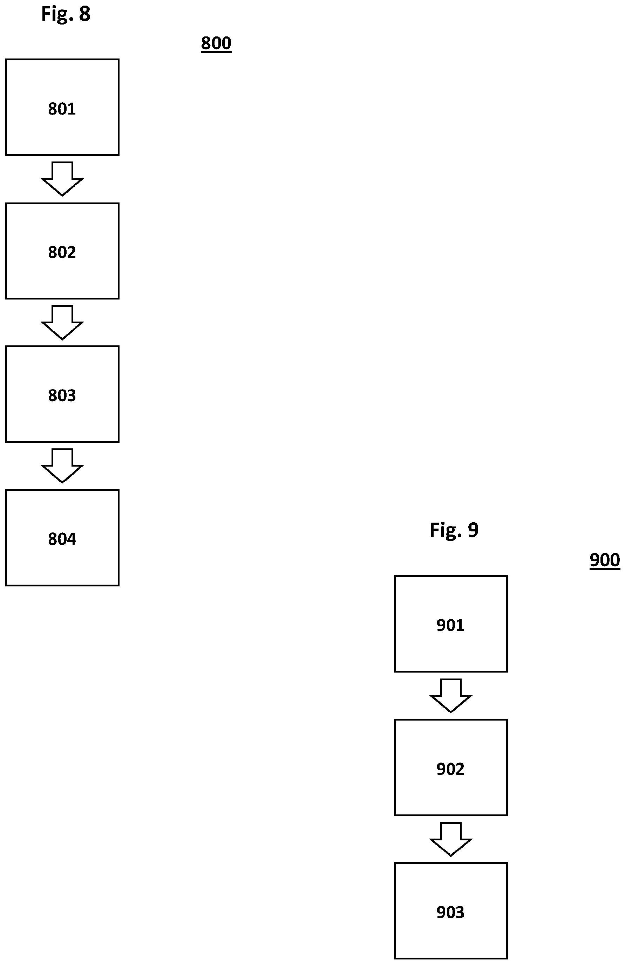

Fig. 8 is a flow diagram showing the steps of a method 800 for filling a plurality of containers with a composition. In step 801 a structure according to the invention is provided (e.g., any of the structures as shown in Figs 5 to 7). In step 802 a plurality of containers is inserted into the structure. This leads to a deformation (moving of the further ends) of the protrusions partially along an insertion direction of the container. This allows for an improved centring of the containers in said receptacles. Once the containers have been received in the receptacles, the further ends of the protrusions return to the position that they had prior to the containers being inserted (the neutral position). Preferably, the protrusions contact the containers such that the containers are not gripped by said protrusions. In step 803 the containers are partially filled with a pharmaceutical composition. In step 804, the partially filled containers are removed from the structure. During removal of the containers, the protrusions deform (moving of the further ends) in a direction that is at least partially along the direction of removal. Once the containers have been removed, the further ends of the protrusion return to the position that they had prior to the containers being inserted (the neutral position).

Fig. 9 is flow diagram showing the steps of a method 900 for a method for transporting a plurality of containers. In step 901 a structure according to the invention is provided (e.g., any of the structures as shown in Figs 5 to 7). In step 902, a plurality of containers is inserted into the structure. Step 902 is as described for step 802 of Fig. 8. In step 903, the structure and the containers received therein are transported from a first location to a further location. A first location may be a sterilisation station to sterilise the structure and the containers received therein. A further location may be a filling station to partially fill the containers with a composition.

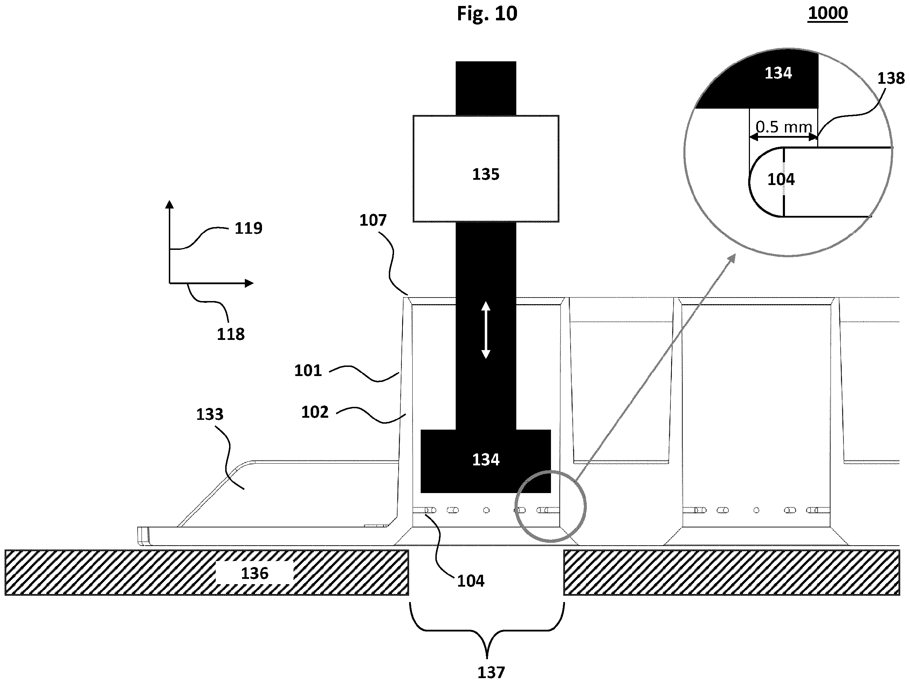

Fig. 10 is a schematic illustration of the test method 1000 used to measure the force required to remove a container from a receptacle. A structure 133 is placed on a support 136 (such as a table). The support 136 has at least one opening 137 that aligns with an opening of a receptacle 101 of the structure 133. A piston 134, attached to a force sensor 135, is inserted into the receptacle 101 and brough into contact with the protrusions. The further ends of the protrusions should not be brought in motion as a result of said contact. Furthermore, the piston 134 is arranged such that a distance of overlap 138 between an edge of the piston and a protrusion 104 is 0.5 mm. The force exerted by the piston 134 on the protrusions 104 is then increased until the further ends of the protrusions 104 move from the neutral position. The force required to bring about said movement is defined as the force required to remove a container from a receptacle.

Although, e.g., Fig. 1 and Figs 5A to 5C show that the protrusions are arranged in a plane that is perpendicular to the further direction (e.g., perpendicular to a length of the receptacles), this is not a requirement. For example, the protrusion may also be arranged in a plane that makes an angle with respect to at least one or all of the following: the first direction, the further direction, an even-further direction that is arranged perpendicular to both the first and further directions.

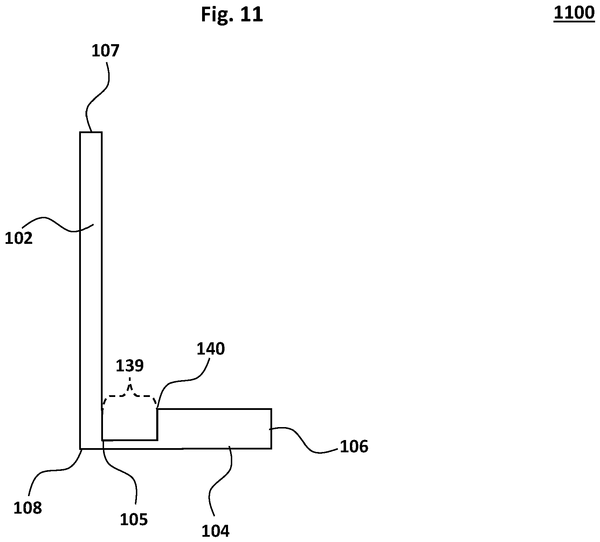

Fig. 11 is an example of a first end 105 of a protrusion 104 that is formed as a hinge. The protrusion 104 has a portion 139 that has a reduced thickness compared to the thickness of the rest of the protrusion 104. When a first end of a protrusion is arranged as a hinge as shown in Fig 11, a thickness of the first end 105 should not be measured at the position of the portion 139, but, at a position 140 immediately adjacent to said portion 139. In other words, when determining the variation in thickness between the first end and the further end of a protrusions, the thickness measured at the position 140 and the thickness measured at the further end 106 are considered.

## TEST METHODS

The test methods which follow were utilized within the context of the invention. Unless stated otherwise, the measurements were conducted at an ambient temperature of 23 °C, an ambient air pressure of 100 kPa (0.986 atm) and a relative air humidity of 50 %.

Dimensions

The length of a receptacle is measured using a calliper. The distance that a protrusion extends into an interior volume, as well as the thickness and width of a protrusion, are also measured using a calliper.

Weight of a structure

A weight $W$ of a structure is given by the following formula:

$$W = M_S * G,$$

where $Ms$ is the mass of the structure, measured in g, and $G$ = 981 cm/s$^2$ is the gravitational acceleration constant.

Force required to remove container

The force required to remove a container from a receptacle is described in Fig. 10 and the accompanying figure description.

Modulus of elasticity

The modulus of elasticity is determined according to the standard ISO 527.

Diameter of a receptacle

A diameter of a receptacle is measured between the two points on a sidewall of the receptacle that are located closest to each other, wherein a line connecting said two points passes through the centre of the receptacle. If a receptacle comprises protuberances (or other structures) that extend into the interior of the receptacle (see, e.g., Fig. 5A), the two closest points may be located on two protuberances (or the other structures).

Angles

Angles are measured using a protractor.

**EXAMPLES**

The invention is illustrated further by way of examples. The invention is not restricted to the examples.

Example 1

Example 1 is repeated with two different structures. In Example 1.1 (an inventive example), a structure as shown in Fig. 5 is provided. Each receptacle has six protrusions arranged at the further ends of said receptacles. Each protrusion extends a distance of 3.5 mm into an interior of a receptacle. The protrusions have a maximum thickness of 1.5 mm. The first ends of the protrusions are arranged as hinges.
In Example 1.2 (a comparative example), a structure similar to the structure of Example 1.1 is provided, with the difference that the structure does not have any protrusions.
For both Examples 1.1 and 1.2, the following steps are performed. Syringes (containers) are inserted into the structure at a first station. The structure and syringes are then transported to a filling station. At the filling station the syringes are partially filled with a pharmaceutical composition. The structure and syringes are then transported to a further station, where the syringes are removed.
Table 1 shows a comparison of the technical effects between Examples 1.1 and 1.2.

**Table 1**

|  | 1.1 | 1.2 |
|---|---|---|
| **Set-up** |  |  |
| Structure has protrusions | Yes | No |
|  |  |  |
| **Technical effect** |  |  |
| Packaging density | ++ | - |
| Reducing tilting of containers | +++ | -- |
| Reducing damage of containers during insertion | + | - |
| Reducing damage of containers during removal | +++ | --- |
| Enhancing the ease of removal of containers | ++ | - |

(continued)

| Technical effect | | |
|---|---|---|
| Reducing damage of containers during transport | ++ | --- |
| Reducing damage to filling needles and containers | + | - |
| Reducing downtime of filling station | ++ | - |
| Reducing particle formation on containers | ++ | - |

The technical effects in Table 1 are as follows:

- Packaging density: the density of syringes in the structure that can be obtained while nevertheless minimising contact between said syringes. A "+" indicates a larger packaging density, and a "-" indicates a lower packaging density. It is desired to increase the packaging density.
- Reducing tilting of containers: how well the syringes are kept upright in the structure during insertion, transportation, and processing (e.g., filling) of the syringes. A "+" indicates that the syringes tilt less, and a "-" indicates that the syringes tilt more. It is desired to have less tilt.
- Reducing damage of containers during insertion: the damage that occurs to syringes during the insertion of the syringes from the structure. A "+" indicates less damage, and a "-" indicates more damage. It is desired to reduce the damage.
- Reducing damage of containers during removal: the damage that occurs to syringes during the removal of the syringes from the structure. A "+" indicates less damage, and a "-" indicates more damage. It is desired to reduce the damage.
- Enhancing the ease of removal of containers: how easily the syringes can be removed from the structure (e.g., the amount of force required). A "+" indicates that it is easier to remove the syringes, and a "-" indicates that it is less easy to remove the syringes. It is desired to increase the ease of removal (e.g., reduce the amount of force required to remove the syringes).
- Reducing damage of containers during transportation: the damage that occurs to syringes during transportation of the syringes in the structure. A "+" indicates less damage, and a "-" indicates more damage. It is desired to reduce the damage.
- Reducing damage to filling needles and containers: when the syringes are partially filled with the composition, contact between the containers and the needles often leads to damage the filling needles and/or the syringes. A "+" indicates less damage, and a "-" indicates more damage. It is desired to reduce the damage.
- Reducing downtime of filling station: downtime as a result of, e.g., problems with the devices used for filling the syringes. A "+" indicates less downtime, and a "-" indicates more downtime. It is desired to decrease the downtime.
- Reducing particle formation on containers: when the syringes are positioned in the structure (such as during transport of the syringes), contact between the receptacle sidewalls and the syringes very often lead to the formation of particles on the surfaces of the syringes. A "+" indicates that less particles are formed, and a "-" indicates that more particles are formed. It is desired to reduce the number of particles formed.

The structures of Examples 1.1 and 1.2 may also be used for, e.g., holding carpules during a filling of said carpules with a composition. Compared to the structure of Example 1.2, the structure of Example 1.1 allows for a significant reduction in the variation of the volumes of the composition in said carpules. It is desired that the volumes of the composition does not vary between carpules.

Example 2

Example 2 is repeated using the structure of Example 1.1, with the following difference. The force required to remove/insert the syringes from the receptacles is varied as shown in Table 2. The weight of the structure is 0.8 N.

**Table 2**

| | 2.1 | 2.2 | 2.3 | 2.4 | 2.5 |
|---|---|---|---|---|---|
| **Set up** | | | | | |
| Force required to insert/remove syringes [N] | 0.05 | 0.1 | 0.3 | 0.9 | 1.1 |
| Reducing damage of containers during insertion/removal | +++ | ++ | + | - | --- |

(continued)

| | 2.1 | 2.2 | 2.3 | 2.4 | 2.5 |
|---|---|---|---|---|---|
| **Set up** | | | | | |
| Reducing damage of structure during insertion | +++ | ++ | - | --- | --- |
| Reducing downtime of process | +++ | ++ | + | - | ---- |
| Reducing particle formation on containers | +++ | ++ | - | -- | --- |

The technical effects in Table 2 are as described for Table 1. In addition, Table 2 has the following additional effects:

- Reducing damage of structure during insertion: the damage to the structure during insertion of the syringes into the structure (insertion very often leads to a deformation of the structure). A "+" indicates less damage to the structure, and a "-" indicates more damage to the structure. It is desired to reduce the damage to the structure during insertion.
- Reducing downtime of process: during the insertion and/or removal of the syringes from the structure, problems may occur with the devices used for the insertion and/or removal of the syringes from the structure. A "+" indicates less downtime, and a "-" indicates more downtime. It is desired to decrease the downtime.

**REFERENCE LIST**

100 Structure
101 Receptacle
102 Sidewall
103 Receptacle interior
104 Protrusion
105 First end of protrusion
106 Further end of protrusion
107 First end of receptacle
108 Further end of receptacle
109 First surface of structure
110 Container
111 First end of container
112 Further end of container
113 Gap between container and sidewall
114 Length of receptacle
115 Distance that protrusion extends into interior of receptacle
116 Thickness of protrusion
117 Width of protrusion
118 First direction
119 Further direction
120 Neutral plane
121 Tangential plane
122 Angle of rotation
123 Axis of rotation
125 Protuberance
126 First set of protrusions
127 Further set of protrusions
128 Distance from first end of receptacle
129 Distance from further end of receptacle
130 Distance between sets of protrusions
131 Body of container
132 Force applied to protrusion
133 Structure
134 Piston

135     Force sensor
136     Support
137     Opening in support
138     Distance of overlap
139     Portion of protrusion
140     Position where thickness is measured

**Claims**

1. A structure comprising a plurality of receptacles, wherein

   a. said receptacles are adapted and arranged for receiving a plurality of containers;
   b. each receptacle, of the plurality of receptacles, comprises a sidewall defining an interior of a respective receptacle, wherein

      i. at least one protrusion is arranged on the sidewall and extends into the interior of the respective receptacle,
      ii. the at least one protrusion has a first end and a further end, wherein

         A. the first end is arranged opposite the further end,
         B. the first end is attached to the sidewall;

   wherein
   at least one section of the further end of the at least one protrusion is adapted and arranged to be movable with respect to the sidewall on which said protrusion is arranged.

2. The structure according to claim 1, wherein at least a segment, preferably the at least one section, of the at least one protrusion, is adapted and arranged to be deformable, preferably elastically deformable.

3. The structure according to any of the preceding claims, wherein the at least one section of the further end is adapted and arranged to rotate through an angle that is in the range from -90 ° to 90 °.

4. The structure according to any of the preceding claims, wherein a force required to remove a container from the respective receptacle is less than or equal to a weight of the structure.

5. The structure according to any of the preceding claims, wherein the force required to remove a container from the respective receptacle is 1 N or less.

6. The structure according to any of the preceding claims, wherein the at least one protrusion has a modulus of elasticity that is in the range 0.80 to 3.50 GPa.

7. The structure according to any of the preceding claims, wherein the at least one protrusion extends a distance $yD_{MIN}$ into the interior volume of the respective receptacle, where $D_{MIN}$ is the minimum diameter of the respective receptacle, where $y$ is in the range from 0.015 to 0.3.

8. The structure according to any of the preceding claims, wherein a maximum thickness of the at least one protrusion is in the range from 0.05 mm to 4 mm.

9. The structure according to any of the preceding claims, wherein the respective receptacle comprises at least two protrusions.

10. The structure according to any of the preceding claims, further comprising a plurality of containers that contain less than 0.1 ml of a composition.

11. The structure according to any of the preceding claims, further comprising a plurality of containers that are at least 20 % filled with a composition, wherein said percentages are with respect to an interior volume of a container.

12. A method for at least partially filling a plurality of containers with a composition, comprising the steps of

**EP 4 635 529 A1**

a. providing a structure according to any of the preceding claims 1 to 11,
b. inserting a plurality of containers in the plurality of receptacles of the structure, wherein the at least one protrusion preferably deforms during said insertion;
c. at least partially filling the containers with the composition.

13. A plurality of containers at least partially filled with a composition, obtainable according to claim 12.

14. A method for transporting a plurality of containers, comprising the steps of

a. providing a structure according to any of the preceding claims 1 to 11;
b. inserting a plurality of containers in the structure;
c. transporting the structure and the plurality of containers from a first location to a further location.

15. A use of a structure according to any of the preceding claims 1 to 11, for at least one or all of the following:

a. reducing contact between the plurality of receptacles and a plurality of containers received in said receptacles;
b. reducing contact between neighbouring containers received in the structure;
c. reducing a particle count on the surfaces of the containers received in the structure;
d. reducing damage to the containers when received in the structure.

18

Fig. 1                                                                                    **100**

**Fig. 2A**                                                                    200

103

101

102                                              102

132

104b

105

104a        106

**Fig. 2B**

103

101

102                                              102

132

104b

105

104a        106

**Fig. 3A**                                                    300

107

115

114

102

103                                                           101

102

105

104

104

116

106

108    104

119

118

**Fig. 3B**

102                    101

117

104

## Fig. 3C

107

102

128

104

129

108

119

118

## Fig. 3D

102

127

130

126

## Fig. 4A

400

102

121

120

122b

122a

104a

106

120

106   104b

## Fig. 4B

121

120

122a

104a

106

119

118

**Fig. 5A**            <u>500</u>

**Fig. 5B**

**Fig. 5C**

**Fig. 6A** <u>600</u>

101

109

104

108

110

112

**Fig. 6B**

109

101

104

## Fig. 6C

**Fig. 7A**    700

**Fig. 7B**

Fig. 7C

**Fig. 8**

800

**Fig. 9**

900

**Fig. 10**

EP 4 635 529 A1

**Fig. 11** <u>**1100**</u>

Europäisches
Patentamt
European
Patent Office
Office européen
des brevets

# EUROPEAN SEARCH REPORT

Application Number

EP 24 17 0194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 136 109 A1 (HOFFMANN LA ROCHE [CH]; ROCHE DIAGNOSTICS GMBH [DE]) 1 March 2017 (2017-03-01) * figures 1-18b * * paragraphs [0002] - [0005], [0008], [0021], [0091], [0094], [0105], [0119], [0168], [0177] - [0185] * | 1-15 | INV. A61M5/00 |
| X | US 2014/034545 A1 (PAWLOWSKI EDGAR [DE] ET AL) 6 February 2014 (2014-02-06) * figures 1-5 * * paragraphs [0046] - [0083] * * claims 1-29 * | 1-15 | |
| X | US 2021/220221 A1 (KOMANN CHRISTIAN [CH] ET AL) 22 July 2021 (2021-07-22) * figures 6-8 * * paragraphs [0009], [0010], [0171] - [0173], [0184] * | 1-15 | |
| X | US 2021/085562 A1 (DEUTSCHLE GREGOR FRITZ [DE]) 25 March 2021 (2021-03-25) * figures 1A-7 * * paragraphs [0010], [0044], [0045], [0073], [0085], [0091] * | 1-15 | **TECHNICAL FIELDS SEARCHED (IPC)** A61M |
| X | US 2003/215365 A1 (SEVIGNY GERARD J [US] ET AL) 20 November 2003 (2003-11-20) * figures 1-18 * * paragraphs [0055], [0062] - [0066] * | 1-15 | |

The present search report has been drawn up for all claims

| Place of search | Date of completion of the search | Examiner |
|---|---|---|
| Munich | 2 October 2024 | López García, Mónica |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 17 0194

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2024

| Patent document cited in search report | | Publication date | Patent family member(s) | | Publication date |
|---|---|---|---|---|---|
| EP 3136109 | A1 | 01-03-2017 | NONE | | |
| US 2014034545 | A1 | 06-02-2014 | NONE | | |
| US 2021220221 | A1 | 22-07-2021 | CN | 113135343 A | 20-07-2021 |
| | | | EP | 3851089 A1 | 21-07-2021 |
| | | | US | 2021220221 A1 | 22-07-2021 |
| US 2021085562 | A1 | 25-03-2021 | CN | 112550910 A | 26-03-2021 |
| | | | CN | 213443868 U | 15-06-2021 |
| | | | EP | 3798142 A1 | 31-03-2021 |
| | | | US | 2021085562 A1 | 25-03-2021 |
| US 2003215365 | A1 | 20-11-2003 | AT | E337097 T1 | 15-09-2006 |
| | | | AU | 2003239485 A1 | 02-12-2003 |
| | | | CA | 2480188 A1 | 27-11-2003 |
| | | | DE | 60307825 T2 | 01-03-2007 |
| | | | EP | 1507594 A2 | 23-02-2005 |
| | | | ES | 2270046 T3 | 01-04-2007 |
| | | | JP | 4235171 B2 | 11-03-2009 |
| | | | JP | 2005526243 A | 02-09-2005 |
| | | | US | 2003215365 A1 | 20-11-2003 |
| | | | US | 2008016969 A1 | 24-01-2008 |
| | | | US | 2008031776 A1 | 07-02-2008 |
| | | | WO | 03097240 A2 | 27-11-2003 |

EPO FORM P0459

For more details about this annex : see Official Journal of the European Patent Office, No. 12/82